# EUROPEAN PATENT SPECIFICATION

(11) **EP 4 093 479 B1**
(45) Date of publication and mention of the grant of the patent: **18.06.2025**
(21) Application number: 21705375.0
(22) Date of filing: 21.01.2021
(51) Int. Cl.: A61M 25/10

(54) **BALLOON PROTECTORS AND BALLOON-CATHETER ASSEMBLIES**
BALLONSCHUTZVORRICHTUNGEN UND BALLONKATHETERANORDNUNGEN
PROTECTEURS DE BALLONNET ET ENSEMBLES CATHÉTER À BALLONNET

(30) Priority: 04.02.2020 US 202062969900 P
(43) Date of publication of application: 30.11.2022
(73) Proprietor: Bard Peripheral Vascular, Inc., Franklin Lakes, NJ 07417 (US)
(72) Inventor: GRISWOLD, David, Ham Lake, MN 55304 (US); LECY, Cyal, Buffalo, MN 55313 (US)
(74) Representative: Hoffmann Eitle
(86) International application number: PCT/US2021/014384
(87) International publication number: WO 2021/158375

(56) References cited:
- WO-A2-2014/179767
- US-A- 6 110 146
- US-A1- 2010 228 333
- US-A1- 2016 058 983
- US-B1- 11 389 627

## Description

### PRIORITY

This application claims the benefit of priority to U.S. Patent Application No. 62/969,900, filed February 4, 2020.

### BACKGROUND

Atherosclerosis is characterized by one or more intravascular lesions formed in part of plaque including blood-borne substances such as fat, cholesterol, and calcium. An intravascular lesion such as an arterial lesion can form on a wall of an arterial lumen and build out across the lumen to an opposite wall thereof. A last point of patency often occurs at a boundary between the arterial lesion and the opposite wall of the arterial lumen. Surgical procedures for atherosclerosis such as balloon angioplasty with drug-coated balloons can be used to restore patency and blood flow lost to the one or more intravascular lesions.

Balloon protectors are designed to cover and protect the drug-coated balloons of balloon catheters during shipping or preoperative handling, which is needed to maintain integrity of the balloon catheters for the surgical procedures for which they are designed. In addition, balloon protectors protect clinicians and others from inadvertently coming into contact with anti-proliferative drugs of the drug-coated balloons when handling the balloon catheters before the surgical procedures. While the foregoing needs for balloon protectors continue to exist, additional needs exist such as a need to safeguard against advancing a balloon catheter through an introducer sheath without first removing the balloon protector thereof.

US 6,110,146 discloses a balloon protector for the balloon of a dilatation catheter including a distally extending flared section that defines a funnel receptive to a guidewire in order to facilitate backloading of the guidewire into the catheter. WO2014/179767 discloses a peelable protective sheath for protecting balloon of angioplasty dilatation catheter for treating blockages in arteries of patient, has tab extending axially from body portion and including textured surface on side facing away from axis.

Disclosed herein are balloon protectors, balloon catheters including the balloon protectors, and methods thereof that meet or exceed the foregoing needs.

### SUMMARY

The present invention is directed to the catheter assembly of claim 1. The dependent claims refer to preferred embodiments.

Disclosed herein is a balloon protector for a balloon catheter. The balloon protector includes a balloon-covering section and a tabbed section proximal of the balloon-covering section. The balloon-covering section is configured to cover a drug-coated balloon of the balloon catheter. The balloon-covering section includes a tip piece of the balloon protector. The tabbed section includes a pair of tabs configured to be continuously pulled away from each other to progressively split the balloon protector along the balloon-covering section until the balloon protector is completely peeled off the balloon.

In some embodiments, the tip piece has a tapered portion having an inner-diameter taper (configured to be) commensurate with an outer diameter of a distal shoulder of the balloon. The inner-diameter taper of the tip piece is configured to maintain concentricity between an opening in a distal end of the tip piece and an opening in a distal end of a shaft of the balloon catheter for threading a guidewire therethrough.

In some embodiments, the tip piece has an overlapping portion over the distal-end portion of the body piece. In other words, the overlapping portion partially overlaps the distal-end portion of the body piece. The overlapping portion has an outer diameter greater than that of an immediately proximal portion of the balloon-covering section. The outer diameter of the overlapping portion of the tip piece is configured to provide a stop to prevent insertion of the balloon catheter into an introducer sheath while the balloon protector is covering the balloon.

In some embodiments, the tip piece includes a longitudinal gap on each side of two opposing sides of the balloon protector. The longitudinal gap has a length greater than or equal to that of the overlapping portion of the tip piece. The balloon protector is configured such that the length of the longitudinal gap allows the distal-end portion of the body piece to be split through a distal end of the body piece without an increase in applied peel force.

In some embodiments, the length of the longitudinal gap is greater than that of the overlapping portion of the tip piece. Due to the length of the longitudinal gap, the balloon protector is configured such that a through hole is formed between a distal end of the longitudinal gap and the distal end of the body piece.

In some embodiments, the balloon protector is configured such that the through hole is configured to provide a break in the applied peel force when splitting the balloon protector along the balloon-covering section, the break signaling splitting through the distal end of the body piece.

In some embodiments, both the body piece and the tip piece of the balloon-covering section include a pair of opposing longitudinal lines of weakness aligned with the tabs. The balloon protector is configured such that the balloon-covering section is configured to split along each line of the lines of weakness.

In some embodiments, the balloon-covering section further includes a transitional section configured to cover a proximal shoulder of the balloon. The transitional section has an outer-diameter flare from an (immediately) distal portion of the balloon-covering section to a distal end of the tabbed section. The outer-diameter flare is configured to increase a distance between the tabs. This is optional for making the tabs easier to grasp.

In some embodiments, each tab of the pair of tabs includes an inner surface and an outer surface. The inner and outer surfaces of each tab are optionally textured. This is optional for making the tabs easier to grasp.

The catheter assembly of claim 1 includes a balloon catheter and a balloon protector. The balloon catheter includes a shaft and a drug-coated balloon in an uninflated state disposed about a distal-end portion of the shaft. The balloon protector can be the balloon protector of any of the foregoing paragraphs [0004] to [0012]. The balloon protector includes a balloon-covering section and a tabbed section proximal of the balloon-covering section. The balloon-covering section covers the balloon of the balloon catheter. The balloon-covering section includes a tip piece of the balloon protector coupled to a distal-end portion of a body piece of the balloon protector. The tabbed section includes a pair of tabs configured to be continuously pulled away from each other to progressively split the balloon protector along the balloon-covering section until the balloon protector is completely peeled off the balloon.

According to claim 1, the tip piece has a tapered portion having an inner-diameter taper commensurate with an outer diameter of a distal shoulder of the balloon. The inner-diameter taper of the tip piece is configured to maintain concentricity between an opening in a distal end of the tip piece and an opening in a distal end of a shaft of the balloon catheter for threading a guidewire therethrough.

In some embodiments, the catheter assembly further includes a stylet disposed in a guidewire lumen of the shaft of the balloon catheter such that the catheter assembly is configured to maintain integrity of the catheter assembly during shipping or preoperative handling. The stylet has a coiled distal-end portion extending from the opening in the distal end of the tip piece of the balloon protector configured to be grasped for pulling the stylet out of the catheter assembly.

In some embodiments, the tip piece has an overlapping portion over the distal-end portion of the body piece. The overlapping portion has an outer diameter greater than that of an immediately proximal portion of the balloon-covering section. The outer diameter of the overlapping portion of the tip piece is configured to provide a stop to prevent insertion of the balloon catheter into an introducer sheath while the balloon protector is covering the balloon.

In some embodiments, the tip piece includes a longitudinal gap on each side of two opposing sides of the balloon protector. The longitudinal gap has a length greater than or equal to that of the overlapping portion of the tip piece. The length of the longitudinal gap allows the distal-end portion of the body piece to be split through a distal end of the body piece without an increase in applied peel force.

In some embodiments, the length of the longitudinal gap is greater than that of the overlapping portion of the tip piece. Due to the length of the longitudinal gap, a through hole is formed between a distal end of the longitudinal gap and the distal end of the body piece.

In some embodiments, the through hole is configured to provide a break in the applied peel force when splitting the balloon protector along the balloon-covering section, the break signaling splitting through the distal end of the body piece.

In some embodiments, both the body piece and the tip piece of the balloon-covering section include a pair of opposing longitudinal lines of weakness aligned with the tabs. The balloon-covering section is configured to split along each line of the lines of weakness.

In some embodiments, the balloon-covering section further includes a transitional section configured to cover a proximal shoulder of the balloon. The transitional section has an outer-diameter flare from an immediately distal portion of the balloon-covering section to a distal end of the tabbed section. The outer-diameter flare is configured to increase a distance between the tabs making the tabs easier to grasp.

In some embodiments, each tab of the pair of tabs includes an inner surface and an outer surface. The inner and outer surfaces of each tab are optionally textured making the tabs easier to grasp.

In some embodiments, the balloon catheter further includes a bifurcated hub about a proximal-end portion of the shaft. The bifurcated hub has an inflation port fluidly connected to the balloon by way of an inflation lumen of the shaft.

Also disclosed herein is a method of a catheter assembly including, in some embodiments, a removing step of removing the catheter assembly from a catheter-assembly package. The catheter assembly can be a catheter assembly of any of the preceding paragraphs [0013] to [0023]. For example, the catheter assembly includes a balloon catheter, a balloon protector, and a stylet. The balloon catheter includes a shaft having a guidewire lumen and a drug-coated balloon in an uninflated state disposed about a distal-end portion of the shaft. The balloon protector can be a balloon protector of any of the preceding paragraphs [0004] to [0012]. For example, the balloon protector includes a balloon-covering section covering the balloon. The balloon-covering section includes a tip piece of the balloon protector coupled to a distal-end portion of a body piece of the balloon protector. The stylet is disposed in the guidewire lumen of the shaft of the balloon catheter. The stylet has a coiled distal-end portion extending from an opening in a distal end of the tip piece of the balloon protector. The method also includes a pulling step of pulling the stylet out of the catheter assembly by the coiled distal-end portion. The method also includes a threading step of threading a proximal-end portion of a guidewire through the opening in the distal end of the tip piece of the balloon protector and into the guidewire lumen of the balloon catheter.

In the invention, the tip piece of the balloon protector has a tapered portion having an inner-diameter taper commensurate with an outer diameter of a distal shoulder of the balloon. The inner-diameter taper of the tip piece is configured to maintain concentricity between the opening in the distal end of the tip piece and an opening in a distal end of the shaft of the balloon catheter. Maintaining concentricity facilitates the threading of the guidewire into the guidewire lumen of the balloon catheter.

In some examples, a distal-end portion of the guidewire is disposed in a blood vessel of a patient about a narrowed or obstructed portion of the blood vessel in need of balloon angioplasty.

In some examples, the method further includes a balloon-catheter advancing step of advancing the balloon catheter over the guidewire to the narrowed or obstructed portion of the blood vessel in need of balloon angioplasty. The method also includes an inflating step of inflating the balloon to widen the narrowed or obstructed portion of the blood vessel. The balloon catheter includes a bifurcated hub over a proximal-end portion of the shaft including an inflation port fluidly connected to the balloon by way of an inflation lumen of the shaft.

In some examples, the method further includes a catheter-assembly advancing step of advancing the catheter assembly over the guidewire up to an opening in a proximal end of an introducer sheath. The tip piece of the balloon protector has an overlapping portion over the distal-end portion of the body piece with an outer diameter greater than that of an immediately proximal portion of the balloon-covering section. The overlapping portion is configured to stop the advancing of the catheter assembly into the introducer sheath with the balloon protector covering the balloon.

In some examples, the method further includes a peeling step of peeling the balloon protector off the balloon catheter by continuously pulling a pair of tabs away from each other to progressively split the balloon protector. The balloon protector includes a tabbed section proximal of the balloon-covering section including the pair of tabs.

In some examples, both the body piece and the tip piece of the balloon-covering section of the balloon protector include a pair of opposing longitudinal lines of weakness aligned with the tabs. The balloon-covering section is configured to split along each line of the lines of weakness.

These and other features of the concepts provided herein will become more apparent to those of skill in the art in view of the accompanying drawings and following description, which disclose particular embodiments of such concepts in greater detail.

### DRAWINGS

FIG. 1 illustrates a side view of a balloon protector in accordance with some embodiments.
FIG. 2 illustrates a top or bottom view of the balloon protector in accordance with some embodiments.
FIG. 3 illustrates a detailed side view of a distal-end portion of the balloon protector in accordance with some embodiments.
FIG. 4 illustrates a detailed top or bottom view of the distal-end portion of the balloon protector in accordance with some embodiments.
FIG. 5 illustrates a longitudinal cross section of the distal-end portion of the balloon protector in accordance with the invention.
FIG. 6 illustrates a transverse cross section of the distal-end portion of the balloon protector where a tip piece overlaps a distal-end portion of a body piece in accordance with some embodiments.
FIG. 7 illustrates a transverse cross section of a distal-end portion of the tip piece of the balloon protector in accordance with some embodiments.
FIG. 8 illustrates an exemplary balloon catheter.
FIG. 9 illustrates a catheter assembly including the balloon catheter and the balloon protector in accordance with some embodiments.
FIG. 10 illustrates a detailed side view of the catheter assembly including a stylet in accordance with some embodiments.
FIG. 11 illustrates a detailed side view of an existing catheter assembly including a stylet in accordance with some examples.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "front," "back," "top," "bottom," "proximal," "distal," and the like, are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

Again, balloon protectors are designed to cover and protect the drug-coated balloons of balloon catheters during shipping or preoperative handling, which is needed to maintain integrity of the balloon catheters for the surgical procedures for which they are designed. In addition, balloon protectors protect clinicians and others from inadvertently coming into contact with anti-proliferative drugs of the drug-coated balloons when handling the balloon catheters before the surgical procedures. While the foregoing needs for balloon protectors continue to exist, additional needs exist such as a need to safeguard against advancing a balloon catheter through an introducer sheath without first removing the balloon protector thereof.

Disclosed herein are balloon protectors, balloon catheters including the balloon protectors, and methods thereof that meet or exceed the foregoing needs.

### Balloon protectors

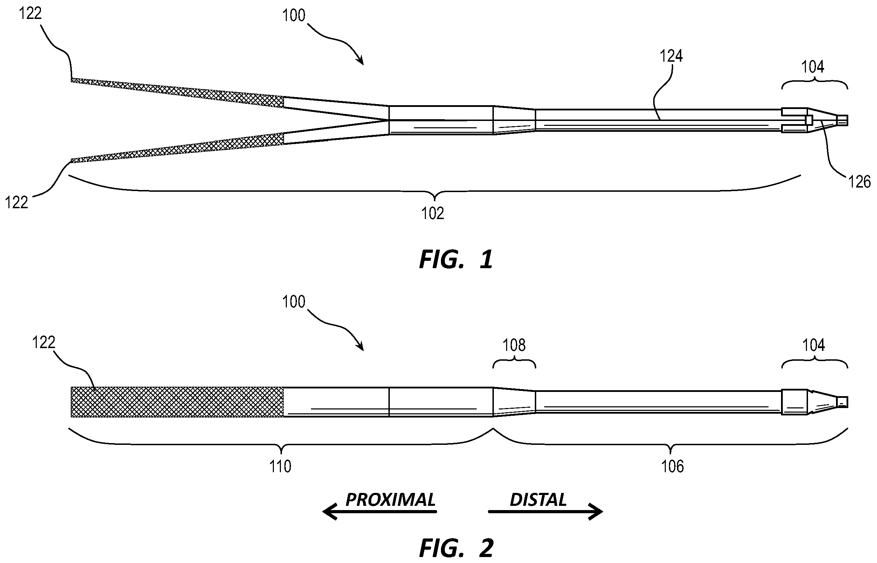

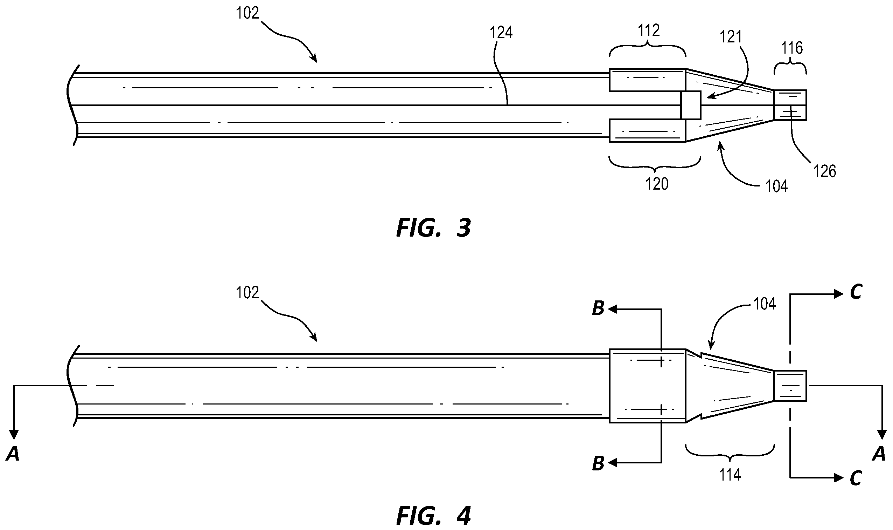

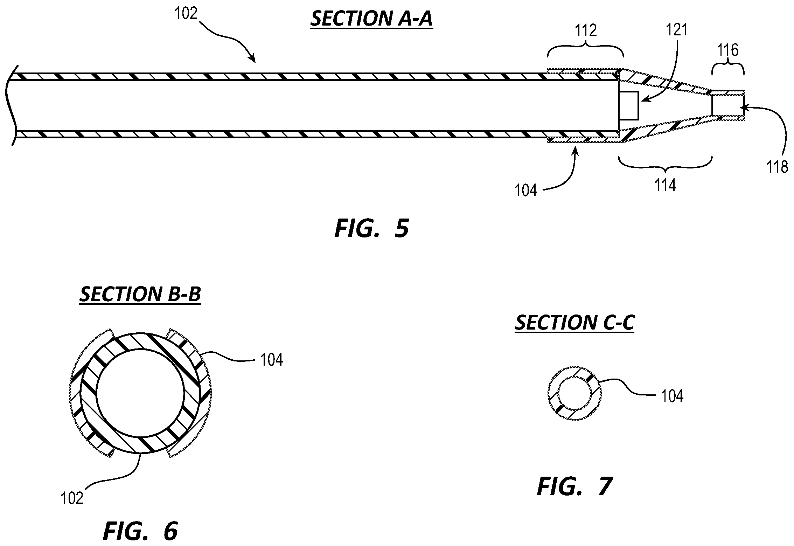

FIGS. 1 and 2 illustrate side and top or bottom views of a balloon protector 100 in accordance with some embodiments. FIGS. 3 and 4 illustrate detailed side and top or bottom views of a distal-end portion of the balloon protector 100 in accordance with some embodiments. FIG. 5 illustrates a longitudinal cross section of the distal-end portion of the balloon protector 100; FIG. 6 illustrates a transverse cross section of the distal-end portion of the balloon protector where a tip piece 104 overlaps a distal-end portion of a body piece 102; and FIG. 7 illustrates a transverse cross section of a distal-end portion of the tip piece 104 of the balloon protector 100 in accordance with some embodiments.

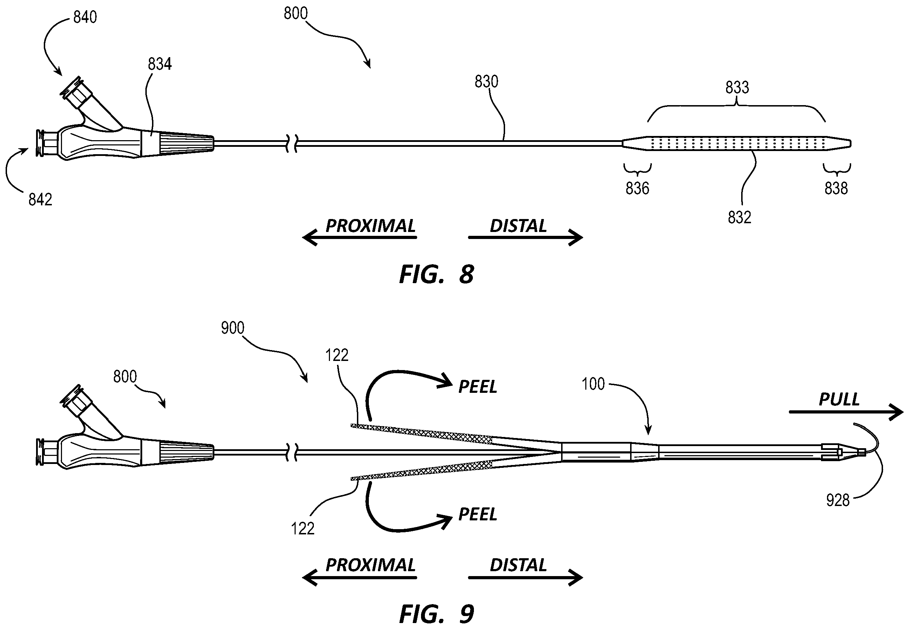

The balloon protector 100 is configured to cover and protect a drug-coated balloon of a balloon catheter (e.g., the balloon catheter 800 of FIG. 8) during shipping or preoperative handling, which is needed to maintain integrity of the balloon catheter for the surgical procedures for which it is designed.

As shown, the balloon protector 100 includes the body piece 102 and the tip piece 104 coupled to a distal-end portion of the body piece 102. Each piece of the body piece 102 and the tip piece 104 is unitary in that it is one continuous piece. A balloon-covering section 106 of the balloon protector 100 includes the tip piece 104 and a distal portion of the body piece 102. The balloon-covering section 106 also includes a transitional section 108 of the body piece 102 opposite the tip piece 104. A tabbed section 110 of the balloon protector 100 includes a proximal portion of the body piece 102.

The balloon-covering section 106 is configured to cover a balloon of a balloon catheter, which balloon is typically disposed about a distal-end portion of a shaft of the balloon catheter. The balloon-covering section 106 is configured to cover the balloon of such a balloon catheter with at least a clearance fit (e.g., a sliding fit, location fit, etc.) up to a transition fit (e.g., tight fit), thereby holding the balloon in an uninflated, folded configuration without compressing the balloon up to minimally compressing the balloon. With respect to the clearance fit, the balloon-covering section 106 of the balloon protector 100 has an inner diameter minimally larger than an outer diameter of the balloon in the uninflated, folded configuration. With respect to the transition fit, the inner diameter of the balloon-covering section 106 is negligibly larger than the outer diameter of the balloon in the uninflated, folded configuration. (*See* International Organization for Standardization ["ISO"] 2768 for such tolerances.)

The tip piece 104 of the balloon protector 100 is configured to cover a distal shoulder of a balloon of a balloon catheter, which shoulder typically does not include a drug coating. The tip piece 104 is coupled (e.g., adhered, solvent bonded, heat welded, etc.) to a distal-end portion of the body piece 102 of the balloon protector 100 such that the tip piece 104 has an overlapping portion 112 over the distal-end portion of the body piece 102. The overlapping portion 112 has an outer diameter greater than that of an immediately proximal portion of the balloon-covering section 106 of the balloon protector 100. The outer diameter of the overlapping portion 112 of the tip piece 104 is configured to provide a stop to prevent insertion of the balloon catheter into an introducer sheath while the balloon protector 100 is covering the balloon. The stop is advantageous in that it is preferential to leave a balloon protector over a balloon of a balloon catheter up until the very moment the balloon catheter is inserted into the introducer sheath in order to protect the balloon from fluids (e.g., saline, bodily fluids, etc.), wet instruments, wet hands, or the like. The stop allows a clinician to leave the balloon protector over the balloon of the balloon catheter as long as desired without a risk of inadvertently inserting the balloon catheter into the introducer sheath while the balloon protector 100 is covering the balloon.

The tip piece 104 has a tapered portion 114 tapering from the overlapping portion 112 of the tip piece 104 to a distal tip 116 of the tip piece 104. The tapered portion 114 of the tip piece 104 has an inner-diameter taper commensurate with an outer diameter of a distal shoulder of a balloon of a balloon catheter. The inner-diameter taper of the tapered portion 114 is configured to maintain concentricity between an opening 118 in the distal tip 116 and an opening in a distal end of a shaft of the balloon catheter for threading a guidewire therethrough. The tapered portion 114 also has an outer-diameter taper. Depending upon the outer diameter of the overlapping portion 112 of the tip piece 104, a length of the tip piece 104, or the like, the outer-diameter taper can be greater than, equal to, or less than the inner-diameter taper. The outer-diameter taper is greater than the inner-diameter taper of the balloon protector 100 shown in FIG. 5. As set forth herein below, the tip piece 104 and the tapered portion 114 thereof facilitate stylet removal.

The tip piece 104 includes a longitudinal gap 120 on each side of two opposing sides of the balloon protector 100. The longitudinal gap 120 of the tip piece 104 has a length equal to or greater than that of the overlapping portion 112 of the tip piece 104 as measured from a proximal end of the tip piece 104. The length of the longitudinal gap 120 allows the distal-end portion of the body piece 102 of the balloon protector 100 to be split through a distal end thereof without the increase in applied peel force that would otherwise be required to split through both the overlapping portion 112 of the tip piece 104 and the distal-end portion of the body piece 102 thereunder. The length of the longitudinal gap 120 in the embodiment of the balloon protector 100 shown in at least FIGS. 1, 3, and 5 is greater than that of the overlapping portion 112. In such embodiments, a window or through hole 121 is formed between a distal end of the longitudinal gap 120 and the distal end of the body piece 102. The through hole 121 advantageously provides a momentary break in the applied peel force (e.g., ~1.5 lbf to 0 lbf) when splitting the balloon protector 100 in a distal direction along the balloon-covering section 106 thereof. The break signals the distal end of the body piece 102 has been split through, which, in turn, signals near completion of the splitting with commencement of the splitting through the tip piece 104.

The transitional section 108 of the balloon protector 100 is configured to cover a proximal shoulder of a balloon of a balloon catheter, which shoulder typically does not include a drug coating. The transitional section 108 has a flare in at least an outer diameter thereof from an immediately distal portion of the balloon-covering section 106 of the balloon protector 100 to a distal end of the tabbed section 110 of the balloon protector 100. The flare of the transitional section 108 is configured to increase a distance between each tab of a pair of tabs 122 of the tabbed section 110 of the balloon protector 100, thereby making the tabs 122 easier to grasp for splitting the balloon protector 100. The flare of the transitional section 108 is also configured to facilitate manufacturing of catheter assemblies such as the catheter assembly 900 set forth below. Indeed, the flare of the transitional section 108 ensures the drug coating 833 of the balloon 832 is not unnecessarily scraped off when the balloon protector 100 is disposed over the balloon 832. In addition, the transitional section 108 minimizes any potential for damage of the balloon 832 while in the uninflated state.

The tabbed section 110 of the balloon protector 100 includes the pair of tabs 122, which tabs 122 are configured to provide a starting point from which to split the balloon-covering section 106 of the balloon protector 100 along the pairs of lines of weakness 124 and 126 set forth below. Indeed, the tabs 122 are configured to be grasped and continuously pulled away from each other to progressively split the balloon protector 100 in a distal direction along the balloon-covering section 106 of the balloon protector 100 until the balloon protector 100 is completely peeled off a balloon of a balloon catheter. Like the flare of the transitional section 108 of the balloon protector 100, the tabbed section 110 has a flare configured to increase a distance between each tab of the pair of tabs 122 of the tabbed section 110, thereby making the tabs 122 easier to grasp for splitting the balloon protector 100. In addition, each tab of the pair of tabs 122 includes an inner surface and an outer surface. Any surface of the inner and outer surfaces of each tab or both surfaces of each tab can be textured making the tabs 122 easier to grasp for splitting the balloon protector 100. In the embodiment of the balloon protector 100 shown in at least FIGS. 1, 2, and 9, both the inner surface and the outer surface of each tab of the pair of tabs 122 is textured.

The body piece 102 along the balloon-covering section 106 of the balloon protector 100 includes a pair of opposing longitudinal lines of weakness 124 aligned with the tabs 122, along which the body piece 102 is configured to split. Likewise, the tip piece 104 of the balloon-covering section 106 of the balloon protector 100 includes a pair of opposing longitudinal lines of weakness 126 aligned with the tabs 122, along which the tip piece 104 is configured to split. Notably, each line of weakness of the lines of weakness 124 of the body piece 102 extends into the longitudinal gap 120 of the tip piece 104 through the distal end of the body piece 102. Each line of weakness of the lines of weakness 126 of the tip piece 104 extends from the distal end of the longitudinal gap 120 of the tip piece 104 through a distal end of the distal tip 116 of the tip piece 104. Should the through hole 121 be present in each side of the balloon protector 100, the through holes provide breaks between the lines of weakness 124 and the lines of weakness 126. The lines of weakness 124 and 126 can be scores or perforations along the balloon-covering section 106, wherein the perforations include a series of longitudinal slits akin to a dashed line, a series of through holes akin to a dotted line, or a combination thereof.

In an alternative to the foregoing, the body piece 102 of the balloon protector 100 lacks the pair of lines of weakness 124, the tip piece 104 of the balloon protector 100 lacks the pair of lines of weakness 126, or both the body piece 102 and the tip piece 104 lack their respective pair of lines of weakness. Instead, the body piece 102, the tip piece 104, or each piece of the body piece 102 and the tip piece 104, as the case might be, is formed of a polymeric material in which main chains of the polymeric material are oriented along a length of the body piece 102 or the tip piece 104. The main chains of the polymeric material are configured to facilitate splitting from a starting point provided by, for example, the pair of tabs 122 when pulled away from each other, through a remainder of the body piece 102 or the tip piece 104 without transversely tearing the body piece 102 or the tip piece 104.

Each piece of the body piece 102 and the tip piece 104 of the balloon protector 100 can be of a polymeric material including, but not limited to, high-density polyethylene ("HDPE") or expanded polytetrafluoroethylene ("ePTFE"). When either the body piece 102 or the tip piece 104 is of ePTFE, main chains of the ePTFE can be oriented along the length of the body piece 102 or the tip piece 104 to facilitate splitting thereof without the pair of lines of weakness 124 or 126 as set for the above. When the body piece 102 or the tip piece 104 is molded from the polymeric material, the body piece 102 or the tip piece 104 is characteristic of the molding process used such as injection molding, extrusion molding, compression molding, bladder molding, or the like.

### Catheter assemblies

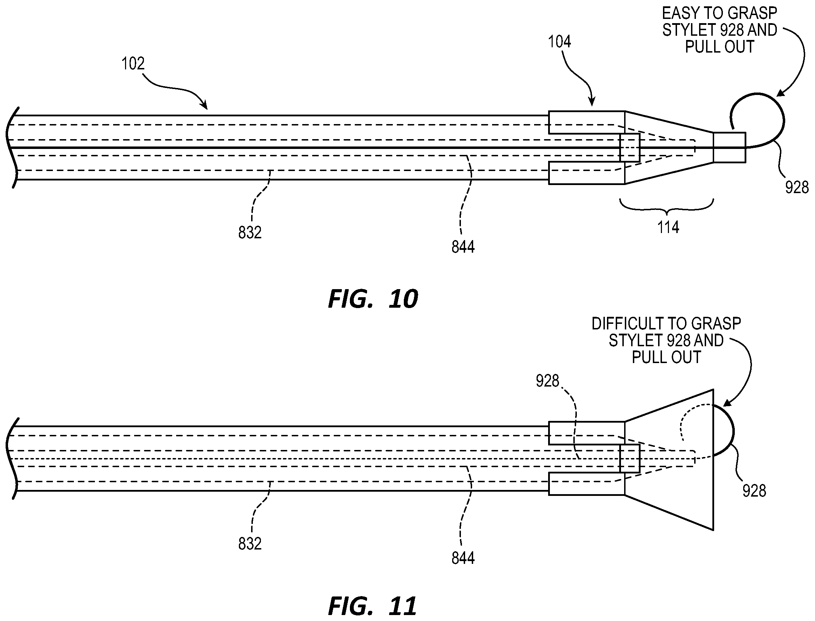

FIG. 8 illustrates a balloon catheter 800 in accordance with some embodiments. FIG. 9 illustrates a catheter assembly 900 including the balloon catheter 800 and the balloon protector 100 in accordance with some embodiments. FIG. 10 illustrates a detailed side view of the catheter assembly 900 including a stylet 928 in accordance with some embodiments.

The catheter assembly 900 includes the balloon catheter 800, the balloon protector 100, and, optionally, the stylet 928 to maintain integrity of the catheter assembly 900 during shipping or preoperative handling.

The balloon catheter 800 includes a shaft 830, a balloon 832 in an uninflated state disposed about a distal-end portion of the shaft 830, and a bifurcated hub 834 about a proximal-end portion of the shaft 830.

The balloon 832 of the balloon catheter 800 can be a drug-coated balloon, wherein a drug coating 833 of the balloon 832 is a coating of an anti-proliferative or anti-restenotic drug such as paclitaxel or rapamycin. The balloon 832 includes a proximal shoulder 836 and a distal shoulder 838, between which shoulders 836 and 838 the drug coating 833 of the balloon 832 is typically applied. That is, the proximal shoulder 836 and the distal shoulder 838 of the balloon 832 do not typically include the drug coating 833. This is because the proximal and distal shoulders 836 and 838 are not typically placed in apposition to an arterial-lumen wall during a surgical intervention for atherosclerosis. Notwithstanding the foregoing, the balloon 832 need not include the drug coating 833 in some embodiments.

The bifurcated hub 834 has an inflation port 840 fluidly connected to the balloon 832 of the balloon catheter 800 by way of an inflation lumen of the shaft 830 of the balloon catheter 800. The bifurcated hub 834 also has a guidewire port 842 connected to a guidewire lumen 844 extending along an entire length of the shaft 830 to an opening in a distal end of a shaft 830. FIGS. 9 and 10 show the stylet 928 within at least a distal-end portion of the guidewire lumen 844 of the shaft 830.

The balloon protector 100 is described as set forth above, which includes details with respect to configuration of the balloon protector 100 over a balloon of a balloon catheter such as the balloon catheter 800, as well as details for how the balloon protector 100 is configured to be removed from such a balloon catheter.

The stylet 928, when present in the catheter assembly 900, is disposed within both the tip piece 104 of the balloon protector 100 and the guidewire lumen 844 of the shaft 830 of the balloon catheter 800 to maintain the integrity of the catheter assembly 900 during shipping or preoperative handling thereof, for example, to prevent collapse of the guidewire lumen 844 of the shaft 830. The stylet 928 can be metal (e.g., stainless steel) with a coiled distal-end portion extending from the opening in the distal tip 116 of the tip piece 104 of the balloon protector 100 configured to prevent the stylet 928 from irretrievably slipping into the guidewire lumen 844 of the shaft 830. The coiled distal-end portion of the stylet 928 is also configured to provide a handle for grasping and pulling the stylet 928 out of the catheter assembly 900 before use of the catheter assembly 900.

As shown by contrast between FIGS. 10 and 11, the tip piece 104 of the balloon protector 100 and the tapered portion 114 thereof advantageously facilitates grasping and pulling the stylet 928 out of the catheter assembly 900, which can be difficult with catheter assemblies such as that of FIG. 11. For example, the catheter assembly of FIG. 11 has a balloon protector with a flared distal-end portion - not a tapered distal-end portion like that of the balloon protector 100. The coiled distal-end portion of the stylet 928 can coil into and conceal itself in the flared distal-end portion of the balloon protector of the catheter assembly of FIG. 11 making it difficult to grasp and pull the stylet 928 out of the catheter assembly. Due to the configuration of the tip piece 104, however, the coiled distal-end portion of the stylet 928 remains exposed, thereby facilitating the grasping and pulling of the stylet 928 out of the catheter assembly 900.

The catheter assembly 900 can be sterilely packaged and ready for immediate use.

### Methods

Methods the catheter assembly 900 include a method of using the catheter assembly 900.

For example, the method of using the catheter assembly 900 includes a removing step of removing the catheter assembly 900 from a package including the catheter assembly 900. For the purpose of this example method, the catheter assembly 900 includes the balloon catheter 800, the balloon protector 100, and the stylet 928. The balloon catheter 800 includes the shaft 830 having the guidewire lumen 844 and the balloon 832 in an uninflated state disposed about the distal-end portion of the shaft 830. The balloon 832 includes the drug coating 833 thereon. The balloon protector 100 includes the balloon-covering section 106 covering the balloon 832. The balloon-covering section 106 includes the tip piece 104 of the balloon protector 100 coupled to the distal-end portion of the body piece 102 of the balloon protector 100. The stylet 928 is disposed in the guidewire lumen 844 of the shaft 830 of the balloon catheter 800 to maintain integrity of the catheter assembly 900 during shipping or preoperative handling. The stylet 928 has the coiled distal-end portion extending from the opening in the distal tip 116 of the tip piece 104 of the balloon protector 100.

The method includes a pulling step of pulling the stylet 928 out of the catheter assembly 900 after the removing step. The pulling step includes pulling the stylet 928 out of the catheter assembly 900 by the coiled distal-end portion of the stylet 928.

The method includes a threading step of threading a proximal-end portion of a guidewire through the opening in the distal tip 116 of the tip piece 104 of the balloon protector 100 and into the guidewire lumen 844 of the balloon catheter 800. The distal-end portion of the guidewire is typically already disposed in a blood vessel of a patient about a narrowed or obstructed portion of the blood vessel in need of balloon angioplasty.

While not expressly shown, the contrast between FIGS. 10 and 11 alludes to how the tip piece 104 of the balloon protector 100 and the tapered portion 114 thereof advantageously facilitates the threading step, which can be difficult with catheter assemblies such as that of FIG. 11. As set forth above, the catheter assembly of FIG. 11 has a balloon protector with a flared distal-end portion - not a tapered distal-end portion like that of the balloon protector 100. The flared distal-end portion of the balloon protector can conceal a distal-end portion of a shaft of a balloon catheter and an opening of a guidewire lumen thereof making the threading step difficult with a lot of poking around. Indeed, clinicians often remove such a balloon protector before it is desired to do so in order to perform the threading step, which risks exposing the balloon of the balloon catheter to fluids (e.g., saline, bodily fluids, etc.), wet instruments, wet hands, or the like. Due to the configuration of the tip piece 104, however, the opening 118 in the distal tip 116 of the tip piece 104 remains exposed, thereby facilitating the threading step (i.e., threading the proximal-end portion of the guidewire through the opening 118 in the distal tip 116 of the tip piece 104 of the balloon protector 100 and into the guidewire lumen 844 of the balloon catheter 800). Of course, the tapered portion 114 of the tip piece 104 has the inner-diameter taper commensurate with the outer diameter of the distal shoulder 838 of the balloon 832, which maintains concentricity between the opening 118 in the distal tip 116 of the tip piece 104 and the opening in the distal end of the shaft 830 of the balloon catheter 800, thereby further facilitating the threading step.

The method includes a catheter-assembly advancing step of advancing the catheter assembly 900 over the guidewire up to an opening in a proximal end of an introducer sheath. As set forth above, the tip piece 104 of the balloon protector 100 has the overlapping portion 112 over the distal-end portion of the body piece 102 of the balloon protector 100 with an outer diameter greater than that of an immediately proximal portion of the balloon-covering section 106 of the balloon protector 100. The overlapping portion 112 is configured to stop the advancing of the catheter assembly 900 into the introducer sheath with the balloon protector 100 covering the balloon 832.

The method includes a peeling step of peeling the balloon protector 100 off the balloon catheter 800 by continuously pulling the pair of tabs 122 of the tabbed section 110 of the balloon protector 100 away from each other to progressively split the balloon protector 100. As set forth above, both the body piece 102 and the tip piece 104 of the balloon-covering section 106 of the balloon protector 100 include the pairs of lines of weakness 124 and 126 aligned with the tabs 122. The balloon-covering section 106 is configured to split along each line of the lines of weakness 124 and 126.

The method can further include a balloon-catheter advancing step of advancing the balloon 832 of the balloon catheter 800 over the guidewire to the narrowed or obstructed portion of the blood vessel in need of balloon angioplasty.

The method can include an inflating step of inflating the balloon 832 to widen the narrowed or obstructed portion of the blood vessel. As set forth above, the balloon catheter 800 includes the bifurcated hub 834 over a proximal-end portion of the shaft 830 including the inflation port 840 fluidly connected to the balloon 832 by way of the inflation lumen of the shaft 830, which inflation port 840 is used for the inflating step.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A catheter assembly (900), comprising:
a balloon catheter (800) including:
a shaft (830); and
a drug-coated balloon (832) in an uninflated state disposed about a distal-end portion of the shaft; and
a balloon protector (100) including:
a balloon-covering section (106) covering the balloon (832), the balloon-covering section (106) including a tip piece (104) of the balloon protector (100) coupled to a distal-end portion of a body piece (102) of the balloon protector (100); and
a tabbed section (110) proximal of the balloon-covering section (106), the tabbed section including a pair of tabs (122) configured to be continuously pulled away from each other to progressively split the balloon protector (100) along the balloon-covering section (106) until the balloon protector (100) is completely peeled off the balloon (832),
wherein the tip piece (104) has a tapered portion (114) having an inner-diameter taper commensurate with an outer diameter of a distal shoulder (838) of the balloon (832), the inner-diameter taper of the tip piece (104) configured to maintain concentricity between an opening in a distal end (116) of the tip piece (104) and an opening in a distal end of the balloon catheter (800) for threading a guidewire therethrough.

2. The catheter assembly of claim 1, further comprising a stylet (928) disposed in a guidewire lumen (844) of the shaft (830) of the balloon catheter (800) to maintain integrity of the catheter assembly (900) during shipping or preoperative handling, the stylet (928) having a coiled distal-end portion extending from the opening in the distal end (116) of the tip piece (104) of the balloon protector configured to be grasped for pulling the stylet (928) out of the catheter assembly (900).

3. The catheter assembly of any claim of claims 1-2, wherein the tip piece (104) has an overlapping portion (112) over the distal-end portion of the body piece (102) having an outer diameter greater than that of an immediately proximal portion of the balloon-covering section (112), the outer diameter of the overlapping portion (112) of the tip piece (104) configured to provide a stop to prevent insertion of the balloon catheter (800) into an introducer sheath while the balloon protector (100) is covering the balloon.

4. The catheter assembly of claim 3, wherein the tip piece (104) includes a longitudinal gap (120) on each side of two opposing sides of the balloon protector (100), the longitudinal gap (120) having a length greater than or equal to that of the overlapping portion (112) of the tip piece (104) allowing the distal-end portion of the body piece to be split through a distal end of the body piece (102) without an increase in applied peel force.

5. The catheter assembly of claim 4, wherein the length of the longitudinal gap (120) is greater than that of the overlapping portion (112) of the tip piece (104), thereby forming a through hole (121) between a distal end of the longitudinal gap (120) and the distal end of the body piece (102).

6. The catheter assembly of claim 5, wherein the through hole (121) is configured to provide a break in the applied peel force when splitting the balloon protector (100) along the balloon-covering section, the break signaling splitting through the distal end of the body piece (102).

7. The catheter assembly of any claim of claims 1-6, wherein both the body piece (102) and the tip piece (104) of the balloon-covering section include a pair of opposing longitudinal lines of weakness (126) aligned with the tabs (122), the balloon-covering section configured to split along each line of the lines of weakness (126).

8. The catheter assembly of any claim of claims 1-7, the balloon-covering section (106) further including a transitional section (108) configured to cover a proximal shoulder (836) of the balloon, the transitional section (108) having an outer-diameter flare from an immediately distal portion of the balloon-covering section (106) to a distal end of the tabbed section (110) configured to increase a distance between the tabs (122) making the tabs easier to grasp.

9. The catheter assembly of any claim of claims 1-8, wherein each tab (122) of the pair of tabs includes an inner surface and an outer surface, the inner and outer surfaces of each tab (122) are textured including making the tabs easier to grasp.

10. The catheter assembly of any claim of claims 1-9, wherein the balloon catheter (800) further includes a bifurcated hub (834) about a proximal-end portion of the shaft, the bifurcated hub (834) having an inflation port (840) fluidly connected to the balloon by way of an inflation lumen of the shaft (830).

## Patentansprüche

1. Katheteranordnung (900), umfassend:
einen Ballonkatheter (800), einschließlich:
eines Schafts (830); und
eines arzneimittelbeschichteten Ballons (832) in einem nicht aufgeblasenen Zustand, der um einen distalen Endabschnitt des Schafts herum angeordnet ist; und
einer Ballonschutzvorrichtung (100), einschließlich:
eines Ballonabdeckabschnitts (106), der den Ballon (832) abdeckt, wobei der Ballonabdeckabschnitt (106) ein Spitzenteil (104) der Ballonschutzvorrichtung (100) einschließt, das mit einem distalen Endabschnitt eines Körperteils (102) der Ballonschutzvorrichtung (100) gekoppelt ist; und
eines Laschenabschnitts (110), proximal zu dem Ballonabdeckabschnitt (106), wobei der Laschenabschnitt ein Paar von Laschen (122) einschließt, die dafür konfiguriert sind, beständig voneinander weg gezogen zu werden, um die Ballonschutzvorrichtung (100) entlang des Ballonabdeckabschnitts (106) fortschreitend zu teilen, bis die Ballonschutzvorrichtung (100) vollständig von dem Ballon (832) abgelöst ist,
wobei das Spitzenteil (104) einen verjüngten Abschnitt (114) mit einer Verjüngung eines Innendurchmessers aufweist, die einem Außendurchmesser einer distalen Schulter (838) des Ballons (832) entspricht, wobei die Verjüngung des Innendurchmessers des Spitzenteils (104) dafür konfiguriert ist, eine Konzentrität zwischen einer Öffnung in einem distalen Ende (116) des Spitzenteils (104) und einer Öffnung in einem distalen Ende des Ballonkatheters (800) beizubehalten, um einen Führungsdraht hindurch zu ziehen.

2. Katheteranordnung nach Anspruch 1, weiter umfassend ein Stilett (928), das in einem Führungsdrahtlumen (844) des Schafts (830) des Ballonkatheters (800) angeordnet ist, um eine Integrität der Katheteranordnung (900) während Versand oder Handhabung vor dem Betrieb beizubehalten, wobei das Stilett (928) einen gewundenen distalen Endabschnitt aufweist, der sich von der Öffnung in dem distalen Ende (116) des Spitzenteils (104) der Ballonschutzvorrichtung erstreckt und dafür konfiguriert ist, ergriffen zu werden, um das Stilett (928) aus der Katheteranordnung (900) herauszuziehen.

3. Katheteranordnung nach einem der Ansprüche 1-2, wobei das Spitzenteil (104) einen Überlappungsabschnitt (112) über den distalen Endabschnitt des Körperteils (102) mit einem Außendurchmesser aufweist, der größer als jener eines unmittelbar proximalen Abschnitts des Ballonabdeckabschnitts (112) ist, wobei der Außendurchmesser des Überlappungsabschnitts (112) des Spitzenteils (104) zum Bereitstellen eines Anschlags konfiguriert ist, um eine Einführung des Ballonkatheters (800) in eine Einführhülse zu verhindern, während die Ballonschutzvorrichtung (100) den Ballon abdeckt.

4. Katheteranordnung nach Anspruch 3, wobei das Spitzenteil (104) einen Längsspalt (120) an jeder Seite von zwei entgegengesetzten Seiten der Ballonschutzvorrichtung (100) einschließt, wobei der Längsspalt (120) eine Länge aufweist, die größer als jene oder gleich jener des Überlappungsabschnitts (112) des Spitzenteils (104) ist, sodass der distale Endabschnitt des Körperteils durch ein distales Ende des Körperteils (102) ohne eine Erhöhung der aufgebrachten Ablösungskraft geteilt werden kann.

5. Katheteranordnung nach Anspruch 4, wobei die Länge des Längsspalts (120) größer als jene des Überlappungsabschnitts (112) des Spitzenteils (104) ist, wodurch eine Durchgangsbohrung (121) zwischen einem distalen Ende des Längsspalts (120) und dem distalen Ende des Körperteils (102) gebildet wird.

6. Katheteranordnung nach Anspruch 5, wobei die Durchgangsbohrung (121) dafür konfiguriert ist, eine Unterbrechung der aufgebrachten Ablösungskraft bereitzustellen, wenn die Ballonschutzvorrichtung (100) entlang des Ballonabdeckabschnitts geteilt wird, wobei die Unterbrechung ein Teilen durch das distale Ende des Körperteils (102) signalisiert.

7. Katheteranordnung nach einem der Ansprüche 1-6, wobei sowohl das Körperteil (102) als auch das Spitzenteil (104) des Ballonabdeckabschnitts ein Paar von entgegengesetzten Schwächungslinien (126) in der Längsrichtung einschließen, die mit den Laschen (122) ausgerichtet sind, wobei der Ballonabdeckabschnitt dafür konfiguriert ist, sich entlang jeder Linie der Schwächungslinien (126) zu teilen.

8. Katheteranordnung nach einem der Ansprüche 1-7, wobei der Ballonabdeckabschnitt (106) weiter einen Übergangsabschnitt (108) einschließt, der dafür konfiguriert ist, eine proximale Schulter (836) des Ballons abzudecken, wobei der Übergangsabschnitt (108) eine Aufweitung eines Außendurchmessers von einem unmittelbar distalen Abschnitt des Ballonabdeckabschnitts (106) zu einem distalen Ende des Laschenabschnitts (110) aufweist, die dafür konfiguriert ist, einen Abstand zwischen den Laschen (122) zu vergrößern, sodass die Laschen einfacher zu ergreifen sind.

9. Katheteranordnung nach einem der Ansprüche 1-8, wobei jede Lasche (122) des Paars von Laschen eine Innenfläche und eine Außenfläche einschließt, wobei die Innen- und die Außenfläche jeder Lasche (122) strukturiert sind, einschließlich eines Ermöglichens eines einfacheren Ergreifens der Laschen.

10. Katheteranordnung nach einem der Ansprüche 1-9, wobei der Ballonkatheter (800) weiter einen Gabelungsknotenpunkt (834) um einen proximalen Endabschnitt des Schafts herum einschließt, wobei der Gabelungsknotenpunkt (834) einen Aufblasstutzen (840) in Fluidverbindung mit dem Ballon über ein Aufblaslumen des Schafts (830) aufweist.

## Revendications

1. Ensemble cathéter (900), comprenant :
un cathéter à ballonnet (800) incluant :
un arbre (830) ; et
un ballonnet revêtu de médicament (832) dans un état non gonflé disposé autour d'une partie d'extrémité distale de l'arbre ; et
un protecteur de ballonnet (100) incluant :
une section de recouvrement de ballonnet (106) recouvrant le ballonnet (832), la section de recouvrement de ballonnet (106) incluant une pièce de pointe (104) du protecteur de ballonnet (100) couplée à une partie d'extrémité distale d'une pièce de corps (102) du protecteur de ballonnet (100) ; et
une section à languette (110) située de manière proximale à la section de recouvrement de ballonnet (106), la section à languette incluant une paire de languettes (122) configurées pour être écartées de façon continue l'une de l'autre pour diviser progressivement le protecteur de ballonnet (100) le long de la section de recouvrement de ballonnet (106) jusqu'à ce que le protecteur de ballonnet (100) soit complètement décollé du ballonnet (832),
dans laquelle la pièce de pointe (104) présente une partie effilée (114) présentant un effilement de diamètre interne proportionnel à un diamètre externe d'un épaulement distal (838) du ballonnet (832), l'effilement de diamètre interne de la pièce de pointe (104) étant configuré pour maintenir une concentricité entre une ouverture dans une extrémité distale (116) de la pièce de pointe (104) et une ouverture dans une extrémité distale du cathéter à ballonnet (800) permettant d'enfiler un fil-guide à travers celle-ci.

2. Ensemble cathéter selon la revendication 1, comprenant en outre un stylet (928) disposé dans une lumière de fil-guide (844) de l'arbre (830) du cathéter à ballonnet (800) pour maintenir l'intégrité de l'ensemble cathéter (900) durant une expédition ou une manipulation préopératoire, le stylet (928) présentant une partie d'extrémité distale enroulée s'étendant à partir de l'ouverture dans l'extrémité distale (116) de la pièce de pointe (104) du protecteur de ballonnet configurée pour être saisie de manière à sortir le stylet (928) de l'ensemble cathéter (900).

3. Ensemble cathéter selon l'une quelconque des revendications 1 à 2, dans lequel la pièce de pointe (104) présente une partie chevauchante (112) par-dessus la partie d'extrémité distale de la pièce de corps (102) présentant un diamètre externe supérieur à celui d'une partie immédiatement proximale de la section de recouvrement de ballonnet (112), le diamètre externe de la partie chevauchante (112) de la pièce de pointe (104) étant configuré pour fournir une butée permettant d'empêcher l'insertion du cathéter à ballonnet (800) dans une gaine d'introduction tandis que le protecteur de ballonnet (100) recouvre le ballonnet.

4. Ensemble cathéter selon la revendication 3, dans lequel la pièce de pointe (104) inclut un espace longitudinal (120) sur chaque côté de deux côtés opposés du protecteur de ballonnet (100), l'espace longitudinal (120) présentant une longueur supérieure ou égale à celle de la partie chevauchante (112) de la pièce de pointe (104) permettant à la partie d'extrémité distale de la pièce de corps d'être divisée à travers une extrémité distale de la pièce de corps (102) sans augmentation de la force de décollement appliquée.

5. Ensemble cathéter selon la revendication 4, dans lequel la longueur de l'espace longitudinal (120) est supérieure à celle de la partie chevauchante (112) de la pièce de pointe (104), formant par ce moyen un trou traversant (121) entre une extrémité distale de l'espace longitudinal (120) et l'extrémité distale de la pièce de corps (102).

6. Ensemble cathéter selon la revendication 5, dans lequel le trou traversant (121) est configuré pour fournir une interruption de la force de décollement appliquée lors d'une division du protecteur de ballonnet (100) le long de la section de recouvrement de ballonnet, l'interruption signalant une division à travers l'extrémité distale de la pièce de corps (102).

7. Ensemble cathéter selon l'une quelconque des revendications 1 à 6, dans lequel à la fois la pièce de corps (102) et la pièce de pointe (104) de la section de recouvrement de ballonnet incluent une paire de lignes d'affaiblissement (126) longitudinales opposées alignées avec les languettes (122), la section de recouvrement de ballonnet étant configurée pour se diviser le long de chaque ligne des lignes d'affaiblissement (126).

8. Ensemble cathéter selon l'une quelconque des revendications 1 à 7, la section de recouvrement de ballonnet (106) incluant en outre une section de transition (108) configurée pour recouvrir un épaulement proximal (836) du ballonnet, la section de transition (108) présentant un évasement de diamètre externe depuis une partie immédiatement distale de la section de recouvrement de ballonnet (106) jusqu'à une extrémité distale de la section à languette (110) configuré pour augmenter une distance entre les languettes (122) facilitant la saisie des languettes.

9. Ensemble cathéter selon l'une quelconque des revendications 1 à 8, dans lequel chaque languette (122) de la paire de languettes inclut une surface interne et une surface externe, les surfaces interne et externe de chaque languette (122) étant texturées, incluant le fait de faciliter la saisie des languettes.

10. Ensemble cathéter selon l'une quelconque des revendications 1 à 9, dans lequel le cathéter à ballonnet (800) inclut en outre un raccord bifurqué (834) autour d'une partie d'extrémité proximale de l'arbre, le raccord bifurqué (834) présentant un orifice de gonflage (840) en connexion fluidique avec le ballonnet au moyen d'une lumière de gonflage de l'arbre (830).
